# FASCICULE DE BREVET EUROPEEN

(11) **EP 1 177 799 B1**
(45) Date de publication et mention de la délivrance du brevet: **31.05.2006**
(21) Numéro de dépôt: 01118422.3
(22) Date de dépôt: 31.07.2001
(51) Int. Cl.: A61L 9/04, A61L 9/12

(54) **Diffuseur de produits évaporables**
Spender für verdampfbare Substanzen
Diffuser for volatile products

(30) Priorité: 01.08.2000 FR 0010175
(43) Date de publication de la demande: 06.02.2002
(73) Titulaire: Manka Création SARL, 57500 Saint-Avold (FR)
(72) Inventeur: Wendel, Herbert, 57230 Eguelshardt (FR); Wendel, Serge, 68340 Riquewihr (FR)
(74) Mandataire: Vièl, Christof

(56) Documents cités:
- GB-A- 2 292 083
- US-A- 4 809 912
- US-A- 5 060 858
- US-A- 6 039 266

## Description

L'invention concerne un diffuseur de produits évaporables comprenant un réceptacle contenant le ou les produits devant s'évaporer inclus dans un gel ainsi qu'un dispositif d'évaporation maintenu à la surface d'évaporation du gel pour contraindre celui-ci à conserver une surface d'évaporation pratiquement constante quel que soit le niveau d'évaporation du ou des produits évaporables.

Les diffuseurs de produits évaporables sont couramment utilisés notamment pour parfumer ou purifier l'air d'une pièce, d'une voiture ou d'un placard. En général, le produit évaporable est constitué d'eau additionnée d'un produit gélifiant pour faciliter le maniement du diffuseur et éviter que le produit ne se répande au cas où le réceptacle se reverserait. Le produit évaporable est additionné le plus souvent d'un parfum ainsi que d'une substance destructrice d'odeurs qui a pour fonction d'absorber et de neutraliser les particules odorantes en suspension dans l'air. Il est courant de colorer le mélange d'une couleur rappelant le parfum diffusé.

L'inconvénient de ces diffuseurs de parfums ou de destructeurs d'odeurs réside dans le fait que lors de l'évaporation du produit évaporable, notamment de l'eau, le gel se recroqueville sur lui-même de sorte que la surface d'évaporation diminue considérablement. Il en résulte une diminution notable de l'efficacité de la diffusion du parfum ou de l'absorbeur d'odeur. De surcroît, l'agglomérat qui se forme a un aspect peu séduisant rendant son usage gênant dans un endroit visible.

Le document GB 2 292 083 A présente à la figure 2 un dispositif d'évaporation constitué d'un bol étanche contenant un gel sur lequel est posée une plaque poreuse en terre cuite. Cette plaque, en raison de ses dimensions, ne peut pas pénétrer dans le bol lors de l'évaporation du gel, de sorte que très rapidement elle n'est plus en contact avec la surface d'évaporation de la masse du gel. Le problème précédemment évoqué n'est donc pas résolu.

Pour résoudre ce problème, plusieurs solutions ont été proposées. De US 5,060,858, on connaît un diffuseur de produits évaporables dont le réceptacle est muni à la périphérie de son ouverture de dispositifs d'ancrage formés soit par un matériau poreux soit par des encoches. Le gel en étant partiellement absorbé par le matériau poreux ou en pénétrant dans les encoches est censé présenter une surface d'évaporation stable tout le long de l'évaporation. Cette surface d'évaporation restant toujours proche de l'ouverture du réceptacle, c'est le gel qui en se rétractant remonte dans le récipient.

Une variante d'ancrage du gel près de l'ouverture du réceptacle est connue de US 6,039,266. Ici, le dispositif d'ancrage est constitué d'une grille fixée sur la périphérie de l'ouverture du réceptacle. Lors de la fabrication, la grille est noyée dans la couche superficielle du gel de sorte à être recouverte d'une couche suffisante pour assurer l'ancrage. La surface d'évaporation étant ancrée sur la grille qui est elle-même fixée à la périphérie de l'ouverture du réceptacle, c'est la masse du gel qui remonte dans le réceptacle au cours de l'évaporation.

Ces dispositifs présentent deux inconvénients majeurs. Ils ne peuvent être utilisés qu'avec des gels dont la composition permet la rétraction vers le haut. En effet, si les propriétés mécaniques du gel sont insuffisantes, la masse du gel risque de se disloquer sous son propre poids de sorte que seule une faible partie du gel reste effectivement ancrée au dispositif d'ancrage tandis que le reste de la masse reste au fond du réceptacle. Lorsque la partie supérieure est évaporée, elle forme une sorte de croûte qui gêne considérablement l'évaporation du bloc de gel resté au fond du récipient.

La réalisation de l'ancrage est un second problème pour ce type de diffuseur. En effet, le poids de la masse du gel est relativement important par rapport à la surface d'ancrage disponible. Il faut donc que la couche recouvrant la grille soit suffisante pour assurer l'ancrage, sans quoi l'ensemble de la masse restera au fond du réceptacle et seule la grille restera sur l'ouverture.

Par ailleurs, pour assurer la formation de cette couche superficielle, il est nécessaire tout d'abord de fixer la grille sur l'ouverture principale du récipient, de fermer cette ouverture avec un couvercle, de retourner l'ensemble pour remplir le récipient par une ouverture de remplissage formée à cet effet dans le fond du récipient et enfin de fermer cette ouverture de remplissage avec un bouchon étanche. Ce procédé nécessite donc de nombreuses pièces bien ajustées les unes aux autres sans quoi il y aura des fuites.

L'objectif de l'invention est donc de développer un diffuseur de produits évaporables permettant une évaporation homogène du ou des produits évaporables tout au long de l'utilisation quel que soit le type de gel employé. Le dispositif d'évaporation doit garantir que la masse ne se disloque pas et que la surface d'évaporation du gel reste toujours en contact avec le dispositif d'évaporation. Un second objectif de l'invention est d'obtenir un diffuseur qui nécessite peu de pièces et qui puisse être produit à faibles coûts.

Cet objectif est atteint par un diffuseur selon le préambule dans lequel le dispositif d'évaporation est mobile vers le fond du réceptade durant le mouvement de rétraction vertical du gel lors de l'évaporation. Par conséquent, lors de l'évaporation, le gel se rétracte en entraînant le dispositif d'évaporation. La masse du gel reste toujours au fond du réceptacle et ne risque pas de se disloquer. De plus, il n'y a plus de risque de « désancrage ». Le dispositif d'évaporation est placé et maintenu à la surface d'évaporation du gel durant toute l'évaporation du ou des produits évaporables. On évite ainsi que la rétraction du gel sous l'effet de l'évaporation du ou des produits évaporables ne s'accompagne d'une diminution de la surface d'évaporation. En empêchant le gel de se recroqueviller en un agglomérat peu engageant, le gel conserve un aspect esthétique plus favorable à un usage courant. Par ailleurs, lors de la fabrication, il suffit de verser le gel dans le réceptacle, de placer la grille à la surface et de l'enfoncer dans la couche superficielle pour qu'elle y affleure. Les coûts de production d'un tel diffuseur sont donc beaucoup plus faibles que pour les diffuseurs de l'art antérieur. De plus, on peut mieux surveiller l'avancement de l'évaporation du gel.

Ce dispositif d'évaporation peut être constitué par une plaque présentant plusieurs ouvertures. Cette plaque a de préférence une structure alvéolaire ouverte, formée par exemple par une multitude de petits tubes ouverts à leurs deux extrémités et soudés les uns aux autres par leurs parois. Le gel pénètre ainsi dans les ouvertures de la plaque qui forme une sorte de grille relativement rigide, de sorte qu'au fur et à mesure de l'évaporation des produits évaporables, le gel se rétracte en hauteur tout en conservant une surface d'évaporation constante en raison du maintien imposé par les ouvertures du dispositif d'évaporation.

Il est conforme à l'invention que le gel contenant les produits évaporables comprenne une ou plusieurs substances odoriférantes et / ou un ou plusieurs produits destructeurs d'odeurs et / ou un colorant. Le ou l'un des produits évaporables est de préférence de l'eau. Le diffuseur de produits évaporables selon l'invention est particulièrement adapté à la diffusion de parfum ou de destructeurs d'odeurs.

Un exemple de réalisation privilégié de l'invention est présenté ci-dessous.
- La figure 1: montre une vue schématique du dessus d'un diffuseur selon l'invention ;
- La figure 2: montre une vue schématique en coupe du diffuseur de la figure 1 en début d'utilisation ;
- La figure 3: montre une vue schématique en coupe du diffuseur de la figure 1 en fin d'utilisation ;
- La figure 4: montre schématiquement une vue en coupe d'un diffuseur classique non muni d'un dispositif d'évaporation selon l'invention en fin d'utilisation.

Le diffuseur de produits évaporables selon l'invention est constitué d'un réceptacle (1), en général une boîte circulaire pouvant être fermée par un couvercle non représenté, dans lequel est placé le gel (2) contenant les produits évaporables, les produits odoriférants et les destructeurs d'odeurs. Un dispositif d'évaporation (3) est placé sur la surface d'évaporation du gel. Ce dispositif d'évaporation a pour fonction de contraindre la surface d'évaporation du gel (2) à rester pratiquement constante malgré l'évaporation des produits évaporables.

Un mode de réalisation du dispositif d'évaporation (3) consiste à prendre une plaque présentant plusieurs ouvertures. Le gel pénétrant dans les ouvertures, il se forme dans la surface d'évaporation lors de l'évaporation des sortes de pointes qui se crochètent aux ouvertures contraignant ainsi le gel à conserver sa surface d'évaporation initiale. La disparition de la matière ne se traduit alors que par une diminution de l'épaisseur de la masse de gel.

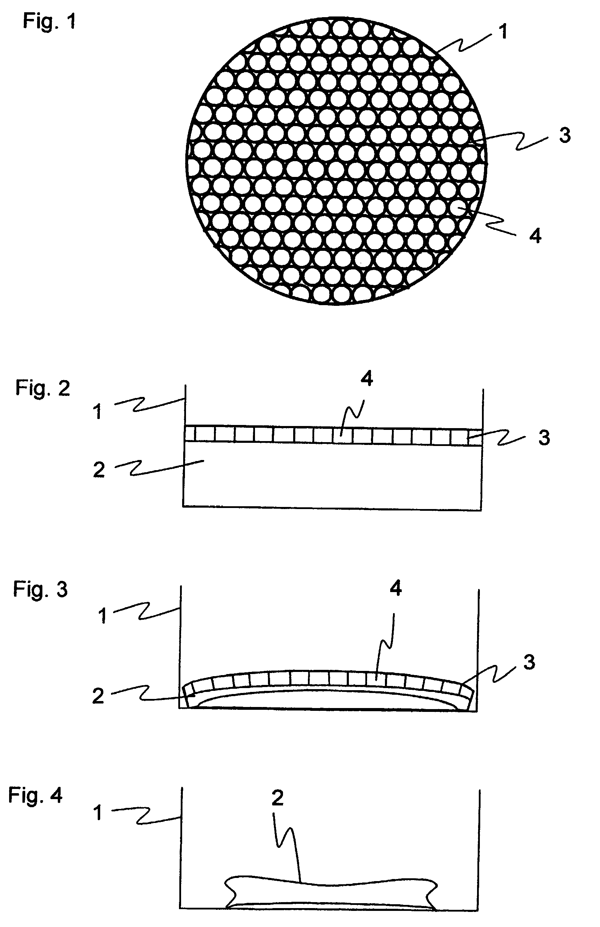

Pour être efficace, la plaque doit présenter une rigidité suffisante pour résister aux forces de rétraction du gel. Afin d'assurer une bonne évaporation des produits évaporables, il est préférable que la surface totale des ouvertures soit aussi proche que possible de la surface potentielle d'évaporation. Autrement dit, il faudrait que la plaque formant le dispositif d'évaporation n'ait qu'une très faible surface intrinsèque. Ceci est obtenu en donnant à la plaque une structure alvéolaire ouverte. Les alvéoles, ouvertes des deux côtés, forment une sorte de grille. Le gel en pénétrant dans toutes les alvéoles forme la surface d'évaporation qui n'est que légèrement inférieure à la surface d'évaporation que pourrait avoir le gel en l'absence de dispositif d'évaporation. Cette structure alvéolaire peut être facilement obtenue en soudant les uns aux autres des petits tubes pour former une grille comme celle représentée à la figure 1. On pourra par exemple utiliser des tubes en matière plastique d'un diamètre d'environ 5,9 mm et d'une hauteur de 5 mm soudés les uns aux autres par leurs parois. Cette solution tout en étant très efficace n'entraîne pas d'augmentation notable du coût de fabrication du diffuseur.

En jouant sur la rigidité du dispositif d'évaporation, il est possible de conserver la forme de la surface d'évaporation initiale, quelle soit plane ou courbe, ou au contraire de permettre un certain cintrage de celle-ci comme cela est montré à la figure 3. Cette modification de la géométrie de la surface d'évaporation n'a cependant pratiquement aucun effet sur la surface totale d'évaporation qui reste sensiblement constante.

La forme du réceptacle n'a aucune importance. Il suffit que le dispositif d'évaporation ait la possibilité de rester en contact avec la surface d'évaporation du gel en suivant librement le mouvement de rétraction vertical du gel lors de l'évaporation. De même, la forme du dispositif d'évaporation peut être librement choisie. Elle épouse de préférence les dimensions approximatives de la section horizontale du réceptacle. La forme des alvéoles peut également être librement choisie.

L'ajout d'un colorant dans la masse du gel peut ajouter une touche esthétique qui peut encore être améliorée en colorant le dispositif d'évaporation et le réceptacle dans des couleurs coordonnées.

En absence de dispositif d'évaporation, la masse de gel se rétracte de tous les côtés pour aboutir à un agglomérat représenté schématiquement à la figure 4 dont l'aspect esthétique est un handicap majeur à sa commercialisation. De plus, la surface d'évaporation diminuant continuellement durant l'usage, son effet diminue au cours du temps.

Grâce au dispositif d'évaporation selon l'invention, on évite tous ces inconvénients en contraignant le gel à conserver approximativement sa surface d'évaporation initiale. De plus, il se forme au fur et à mesure de l'évaporation une sorte de damier ou de structure rappelant les rayons d'une ruche beaucoup plus élégant que l'agglomérat classique. En suivant le mouvement de rétraction vertical de la masse du gel, la masse du gel ne risque pas de se disloquer ni de se désolidariser du dispositif d'évaporation.

Le mode de fabrication du diffuseur est beaucoup plus simple que celui de l'art antérieur. Il suffit de remplir le réceptacle de gel, le placer la grille à la surface et de la faire pénétrer dans la couche superficielle de sorte qu'elle y affleure.

Le diffuseur selon l'invention est donc plus efficace que les diffuseurs de l'art antérieur. Il peut être utilisé avec tout type de gel. Il est facile à produire et ne demande que peut de pièces, ce qui le rend particulièrement économique.

## Revendications

1. Diffuseur de produits évaporables comprenant un réceptacle (1) contenant le ou les produits devant s'évaporer inclus dans un gel (2) ainsi qu'un dispositif d'évaporation (3) maintenu à la surface d'évaporation du gel (2) pour contraindre celui-ci à conserver une surface d'évaporation pratiquement constante quel que soit le niveau d'évaporation du ou des produits évaporables, **caractérisé en ce que** le dispositif d'évaporation (3) est mobile vers le fond du réceptacle (1) durant le mouvement de rétraction vertical du gel lors de l'évaporation.

2. Diffuseur de produits évaporables selon la revendication 1, **caractérisé en ce que** le dispositif d'évaporation (3) est constitué par une plaque présentant plusieurs ouvertures (4).

3. Diffuseur de produits évaporables selon la revendication précédente, **caractérisé en ce que** la plaque (3) présentant plusieurs ouvertures (4) a une structure alvéolaire ouverte.

4. Diffuseur de produits évaporables selon la revendication précédente, **caractérisé en ce que** la structure alvéolaire ouverte est formée par une multitude de petits tubes ouverts à leurs deux extrémités et soudés les uns aux autres par leurs parois.

5. Diffuseur de produits évaporables selon l'une des revendications précédentes, **caractérisé en ce que** le gel (2) comprend une ou plusieurs substances odoriférantes et / ou un ou plusieurs produits destructeurs d'odeurs et / ou un colorant.

6. Diffuseur de produits évaporables selon l'une des revendications précédentes, **caractérisé en ce que** le ou l'un des produits évaporables est de l'eau.

## Claims

1. Diffuser for evaporatable products comprising a receptacle (1) containing the product or products to be evaporated included in a gel (2) and an evaporation device (3) held on the evaporation surface of the gel (2) in order to make the latter keep a practically constant evaporation surface whatever the evaporation level of the evaporatable product or products, **characterised in that** the evaporation device (3) is able to move towards the bottom of the receptacle (1) during the vertical retraction movement of the gel during the evaporation.

2. Diffuser for evaporatable products according to claim 1, **characterised in that** the evaporation device (3) consists of a plate having several openings (4).

3. Diffuser for evaporatable products according to the preceding claim, **characterised in that** the plate (3) having several openings (4) has an open alveolar structure.

4. Diffuser for evaporatable products according to the preceding claim, **characterised in that** the open alveolar structure is formed by a multitude of small tubes open at their two ends and welded to one another by their walls.

5. Diffuser for evaporatable products according to one of the preceding claims, **characterised in that** the gel (2) comprises one or more fragrant substances and/or one or more odour-destroying products and/or a dye.

6. Diffuser for evaporatable products according to one of the preceding claims, **characterised in that** the or one of the evaporatable products is water.

## Patentansprüche

1. Verteiler für verdunstbare Produkte, welcher einen Behälter (1) aufweist, der das oder die zu verdunstenden Produkte in einem Gel (2) eingeschlossen enthält, sowie eine Verdunstungsvorrichtung (3), die an der Verdunstungsoberfläche des Gels (2) gehalten wird, um dieses dazu zu bringen, unabhängig von dem Verdunstungsniveau des oder der verdunstbaren Produkte eine praktisch gleichbleibende Verdunstungsoberfläche beizubehalten, **dadurch gekennzeichnet, dass** die Verdunstungsvorrichtung (3) zu dem Boden des Behälters (1) hin während der senkrechten Rückzugbewegung des Gels bei dem Verdunsten beweglich ist.

2. Verteiler für verdunstbare Produkte gemäß Anspruch 1, **dadurch gekennzeichnet, dass** die Verdunstungsvorrichtung (3) durch eine Scheibe gebildet ist, welche mehrere Öffnungen (4) aufweist.

3. Verteiler für verdunstbare Produkte gemäß dem vorausgegangenen Anspruch, **dadurch gekennzeichnet, dass** die Scheibe (3), welche mehrere Öffnungen (4) aufweist, eine offene wabenartige Struktur hat.

4. Verteiler für verdunstbare Produkte gemäß dem vorausgegangenen Anspruch, **dadurch gekennzeichnet, dass** die offene wabenartige Struktur durch eine Vielzahl kleiner Schläuche gebildet ist, die an ihren beiden Enden geöffnet sind und an ihren Wänden miteinander verschweißt sind.

5. Verteiler für verdunstbare Produkte gemäß einem der vorausgegangenen Ansprüche, **dadurch gekennzeichnet, dass** das Gel (2) eine oder mehrere Duftsubstanzen und/oder ein oder mehrere geruchzerstörende Produkte und/oder ein Farbmittel aufweist.

6. Verteiler für verdunstbare Produkte gemäß einem der vorausgegangenen Ansprüche, **dadurch gekennzeichnet, dass** das oder eins der verdunstbaren Produkte Wasser ist.
